Europäisches Patentamt

European Patent Office

Office européen des brevets

(11) Veröffentlichungsnummer: **0 132 699 B1**

# (12) EUROPÄISCHE PATENTSCHRIFT

(45) Veröffentlichungstag der Patentschrift: **11.09.91**

(51) Int. Cl.⁵: **C07C 17/156**, C07C 19/045

(21) Anmeldenummer: **84108094.8**

(22) Anmeldetag: **11.07.84**

Die Akte enthält technische Angaben, die nach dem Eingang der Anmeldung eingereicht wurden und die nicht in dieser Patentschrift enthalten sind.

(54) **Verfahren zur Erwärmung von Reaktionsluft für die Oxy-Chlorierung von Äthylen.**

(30) Priorität: **20.07.83 DE 3326090**

(43) Veröffentlichungstag der Anmeldung:
**13.02.85 Patentblatt 85/07**

(45) Bekanntmachung des Hinweises auf die Patenterteilung:
**11.09.91 Patentblatt 91/37**

(84) Benannte Vertragsstaaten:
**AT BE CH DE FR GB IT LI LU NL SE**

(56) Entgegenhaltungen: .
**DE-A- 2 442 402**
**DE-A- 3 202 027**
**US-A- 4 031 149**

(73) Patentinhaber: **Uhde GmbH**
**Friedrich-Uhde-Strasse 15 Postfach 262**
**W-4600 Dortmund 1(DE)**

(72) Erfinder: **Link, Gerhard, Dipl.-Ing.**
**Aubachstrasse 55**
**W-6500 Mainz(DE)**
Erfinder: **Rieker, Ulrich**
**Hasselstrasse 49**
**W-6232 Bad Soden(DE)**

(74) Vertreter: **Patentanwälte Meinke und Dabringhaus Dipl.-Ing. J. Meinke Dipl.-Ing. W. Dabringhaus**
**Westenhellweg 67**
**W-4600 Dortmund 1(DE)**

## Beschreibung

Die Erfindung richtet sich auf ein Verfahren zur Erwärmung von Reaktionsluft für die Oxy-Chlorierung von Äthylen unter Druck in einem Oxy-Chlorierungs-Reaktor.

In einem Oxy-Chlorierungs-Reaktor wird Chlorwasserstoff mit Äthylen und Sauerstoff zu Dichloräthan umgesetzt. Die benötigte Reaktionsluft muß erwärmt werden; als Beispiel sei hier ein Temperaturbereich um 135° C bei 5,5 bar angegeben.

Aus der US-A 40 31 149 ist ein Vorheizen der Einspeiseströme bekannt. Weiter zeigt die DE-PS 24 42 402 ein Verfahren, das sich im wesentlichen dadurch auszeichnet, daß dort zur "Kühlung" der Verbrennungsgase aus der Verbrennung von Chlorabfällen mit Luft wasserhaltige Salzsäure aufgegeben wird, mit den mit dieser Verfahrenweise verbundenen erheblichen Nachteile durch die Eingabe von Salzsäure. Zusätzlich wird dort Benzol vor dem Katalysator dem System, und zwar dem Reaktor, zugeführt.

Beide vorgeschlagenen Verfahren bedürfen eines enormen Aufwandes an Apparaten, Wärmetauschern und/oder Pumpen. Zahlreiche äußerst giftige Substanzen, wie Benzol oder Uran-Trioxid, kommen in großer Menge zum Einsatz und führen zu unerwünschten Reaktionsneben- oder Abfallprodukten.

Aufgabe der Erfindung ist die Schaffung einer Lösung, mit der die Erwärmung dieser Reaktionsluft mit einfachen Mitteln erfolgen kann.

Zur Lösung dieser Aufgabe sieht die Erfindung vor, daß die Erwärmung durch heißes Verbrennungsgas einer Rückstandsverbrennung von chlorierten Kohlenwasserstoffen erfolgt, wobei die Reaktionsluft den heißen Verbrennungsgasen in mehreren Teilmengen nacheinander zugeführt wird, wobei die Rückstandsverbrennung und die Zufuhr der zu erwärmenden Reaktionsluft so erfolgt, daß beim Einspeisen in den Oxy-Chlorierungs-Reaktor keine freien Kohlenwasserstoffe in der Reaktionsluft enthalten sind.

Die Erfindung macht sich zunutze, daß in vielen Fällen eine Rückstandsverbrennung von chlorierten Kohlenwasserstoffen zur Verfügung steht, insbesondere z.B. bei VC-Anlagen. Hier greift zur Verhinderung von freien Kohlenwasserstoffen in der Reaktionsluft die Erfindung ein durch das Hinzufügen der Reaktionsluft zu den heißen Verbrennungsgasen in mehreren Teilmengen nacheinander. Damit ist eine besonders optimale Steuerung sowohl der eingesetzten Mengen als auch der zu erreichenden Temperaturen der Reaktionsluft vor dem Oxy-Chlorierung-Reaktor erreichbar.

Der weiteren Optimierung der Verfahrensweise dient der Vorschlag, daß die Rückstandsverbrennung und die Reaktionsluftzufuhr auf dem im wesentlichen gleichen Druckniveau wie demjenigen des Oxy-Chlorierungs-Reaktors erfolgt.

Um zu erreichen, daß den Oxy-Chlorierungs-Reaktor nicht noch chlorierte Kohlenwasserstoffe verlassen, ist nach der Erfindung in Ausgestaltung vorgesehen, daß die chlorierten Kohlenwasserstoffe der Rückstandsverbrennung als Teilmenge dem Oxy-Chlorierungsverfahren entstammen.

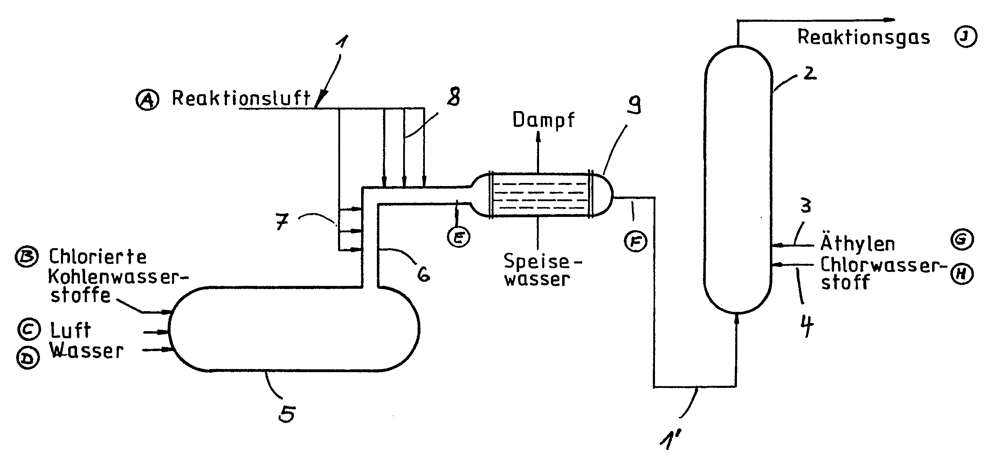

Die Erfindung ist nachstehend anhand der Zeichnung beispielsweise näher erläutert. Diese zeigt ein vereinfacht wiedergegebenes Schema zur Führung des erfindungsgemäßen Verfahrens.

In der Zeichnung sind die eingesetzten Medien angegeben.

Über eine Zuführleitung 1 wird unter Zwischenschaltung weiter unten näher angegebener Elemente einem Oxy-Chlorierungs-Reaktor 2 Reaktionsluft zur Oxy-Chlorierung von Äthylen zugeführt. Die Zuführung des Äthylens zum Reaktor 2 ist mit dem Pfeil 3, die Zuführung von Chlorwasserstoff mit dem Pfeil 4 bezeichnet.

Der Eintritt der "kalten" Reaktionsluft ist im Schaubild links oben mit der Leitung 1 angedeutet. Der Eintritt der erwärmten Reaktionsluft in den Oxy-Chlorierungs-Reaktor trägt die Leitungsbezeichnung 1'.

Zur Angabe von Mengen und Zuständen sind einzelne Punkte im Schaubild mit Großbuchstaben in einem Kreis bezeichnet. So trägt der Eintrittsbereich der Reaktionsluft die Bezeichnung A.

Zur Erwärmung dieser Reaktionsluft wird heißes Verbrennungsgas einer Rückstandsverbrennung herangezogen, das aus einer Brennkammer 5 entnommen wird. Die Entnahmeleitung des heißen Verbrennungsgases ist mit 6 bezeichnet.

Zu dieser Entnahmeleitung 6 führen eine Mehrzahl von Zuführleitungen der Reaktionsluft, deren eines Bündel mit 7 und das andere mit 8 bezeichnet ist, wobei die Darstellung lediglich als Beispiel dient.

Zwischen Eingang in den Oxy-Chlorierungs-Reaktor 2 und dem Ende der Entnahmeleitung 6 ist im dargestellten Beispiel noch ein Wärmetauscher 9 vorgesehen, mit dem noch verfügbare Wärmemengen zur Erzeugung z.B. von Dampf herangezogen werden können. Der Wärmetauscher 9 kann auch entfallen.

Der Eingang der chlorierten Kohlenwasserstoffe in die Brennkammer ist mit "B" bezeichnet, der Eingang der Verbrennungsluft und des Wassers mit "C" bzw. "D". Der Bereich hinter den Zuführleitungen 7 bzw. 8 der Reaktionsluft trägt den Buchstaben "E", der Bereich hinter dem Wärmetauscher 9 den Buchstaben "F". Schließlich sind auch noch die Zuführungen des Äthylens bzw. Chlorwasserstoffes zum Reaktor 2 mit "G" bzw. "H" bezeichnet, während der Ausgang des Reaktionsgases hinter dem Reaktor 2 den Buchstaben "I" trägt. Nachfolgend sind die jeweiligen Mengenangaben lediglich als Beispiel aufgeführt. Einige Temperaturen sind ebenfalls angegeben.

"A":     9.548 kg/h; 25° C

"B":       500 kg/h;

"C":     2.472 kg/h;

"D":       258 kg/h;

"E":   12.805 kg/h; 330° C

"F":            ; 185° C

"G":     2.653 kg/h;

"H":     6.553 kg/h;

"I":    22.011 kg/h.

Die oben angegebenen Mengen und Temperaturen sind lediglich als Beispiel gedacht. Dadurch, daß die Reaktionsluft stufenweise mit dem heißen Abgas aus der Brennkammer 5 vermischt wird, läßt sich eine ganz exakte Prozeßführung erreichen. Diese Zuführleitung 1 bzw. die einzelnen Leitungen der Bereiche 7 und 8 können mit Druckerhöhungsbzw. Druckverminderungseinrichtungen vorgesehen sein, was nicht näher dargestellt ist.

**Patentansprüche**

1. Verfahren zur Erwärmung von Reaktionsluft für die Oxy-Chlorierung von Äthylen unter Druck in einem Oxy-Chlorierungs-Reaktor,
dadurch gekennzeichnet,
daß die Erwärmung durch heißes Verbrennungsgas einer Rückstandsverbrennung von chlorierten Kohlenwasserstoffen erfolgt, wobei die Reaktionsluft den heißen Verbrennungsgasen in mehreren Teilmengen nacheinander zugeführt wird, wobei die Rückstandsverbrennung und die Zufuhr der zu erwärmenden Reaktionsluft so erfolgt, daß beim Einspeisen in den Oxy-Chlorierungs-Reaktor keine freien Kohlenwasserstoffe in der Reaktionsluft enthalten sind.

2. Verfahren nach Anspruch 1,
dadurch gekennzeichnet,
daß die Rückstandsverbrennung und die Reaktionsluftzufuhr auf dem im wesentlichen gleichen Druckniveau wie demjenigen des Oxy-Chlorierungs-Reaktors erfolgt.

3. Verfahren nach Anspruch 1 oder 2,
dadurch gekennzeichnet,
daß die chlorierten Kohlenwasserstoffe der Rückstandsverbrennung als Teilmenge dem Oxy-Chlorierungs-Verfahren entstammen.

**Claims**

1. A method of heating reaction air for the oxychlorination of ethylene under pressure in an oxychlorination reactor characterised in that heating is effected by hot combustion gas from residue combustion of chlorinated hydrocarbons, wherein the reaction air is successively fed to the hot combustion gases in a plurality of quantitative portions, and wherein residue combustion and the feed of the reaction air to be heated are such that no free hydrocarbons are contained in the reaction air upon being fed into the oxychlorination reactor.

2. A method according to claim 1 characterised in that residue combustion and the feed of reaction air occur at substantially the same level of pressure as that in the oxychlorination reactor.

3. A method according to claim 1 or claim 2 characterised in that the chlorinated hydrocarbons in the residue combustion step originate as a portion from the oxychlorination process.

**Revendications**

1. Procédé de chauffage de l'air de réaction pour l'oxychloration de l'éthylène sous pression dans un réacteur d'oxychloration, caractérisé en ce que le chauffage se fait par un gaz de combustion chaud d'une combustion de résidu d'hydrocarbures chlorés, l'air de réaction étant ajouté aux gaz de combustion chauds en plusieurs quantités partielles les unes après les autres, la combustion de résidus et l'addition de l'air de réaction qui doit être chauffé ayant lieu de telle manière que lors de l'introduction dans le réacteur d'oxychloration aucun hydrocarbure libre n'est présent dans l'air de réaction.

2. Procédé selon la revendication 1, caractérisé en ce que la combustion de résidus et l'introduction de l'air de réaction ont lieu sensiblement au même niveau de pression que celui du réacteur d'oxychloration.

3. Procédé selon la revendication 1 ou 2, caractérisé en ce que les hydrocarbures chlorés de la combustion de résidus proviennent du procédé d'oxychloration sous forme de quantité partielle.

Ⓐ Reaktionsluft

Dampf

Reaktionsgas Ⓙ

Ⓑ Chlorierte Kohlenwasser-stoffe

Ⓒ Luft

Ⓓ Wasser

Speise-wasser

Äthylen Ⓖ

Chlorwasser-stoff Ⓗ

EP 0 132 699 B1